Europäisches Patentamt

European Patent Office

Office européen des brevets

(11) Veröffentlichungsnummer: **0 348 782**
**A1**

## (12) EUROPÄISCHE PATENTANMELDUNG

(21) Anmeldenummer: **89111098.3**

(22) Anmeldetag: **19.06.89**

(51) Int. Cl.4: **A61B 1/24**

(30) Priorität: **29.06.88 DE 8808327 U**

(43) Veröffentlichungstag der Anmeldung:
**03.01.90 Patentblatt 90/01**

(84) Benannte Vertragsstaaten:
**AT BE CH DE ES FR GB GR IT LI LU NL SE**

(71) Anmelder: **Henschel, Rolf**
**Herzberger Landstrasse 36a**
**D-3400 Göttingen(DE)**

(72) Erfinder: **Henschel, Rolf**
**Herzberger Landstrasse 36a**
**D-3400 Göttingen(DE)**

(74) Vertreter: **Rehberg, Elmar, Dipl.-Ing.**
**Postfach 3162 Am Kirschberge 22**
**D-3400 Göttingen(DE)**

(54) **Spiegelinstrument, insbesondere Mundspiegel für zahnärztliche Untersuchungen.**

(57) Die Erfindung betrifft einen Mundspiegel für zahnärztliche Untersuchungen, mit einem einen Spiegel aufnehmenden, zumeist kreisförmig ausgebildeten Träger (1) und mit einem mit dem Träger (1) befestigten Handgriff (3), wobei erfindungsgemäß eine Heizvorrichtung (4) vorgesehen ist, die den Spiegel auf insbesondere Körpertemperatur eines Patienten aufheizt.

Fig. 1

EP 0 348 782 A1

## Spiegelinstrument, insbesondere Mundspiegel für zahnärztliche Untersuchungen

Die Erfindung geht aus von einem Spiegelinstrument, insbesondere Mundspiegel für zahnärztliche Untersuchungen, mit einem einen Spiegel aufnehmenden, zumeist kreisförmig ausgebildeten Träger und mit einem mit dem Träger befestigten Handgriff. Ein Spiegelinstrument der eingangs beschriebenen Art ist bekannt. Es dient beispielsweise bei zahnärztlichen Untersuchungen dazu, dem behandelnden Zahnarzt eine freie Sicht auf die Zähne eines Patienten zu ermöglichen. Weiterhin wird das Spiegelinstrument überall dort eingesetzt, wo dem behandelnden Arzt eine freie Sicht auf die zu behandelnde Stelle verwehrt ist, wie dies beispielsweise bei bestimmten Operationen häufig auftritt. Das bekannte Spiegelinstrument weist einen Träger auf, auf dem der eigentliche Spiegel gelagert und befestigt ist. Der Träger ist meist kreisförmig ausgebildet, für bestimmte Anwendungsfälle kann der Träger aber auch andere Formen, beispielsweise oval oder rechteckig, aufweisen. Der Spiegel ist der Form des Trägers entsprechend angepaßt. Um den Spiegel führen zu können, ist an dem Träger ein Handgriff befestigt. Der Träger und der Handgriff des bekannten Spiegelinstruments bestehen zumeist aus Aluminium. Nachteilig bei dem bekannten Spiegelinstrument ist, daß die Gefahr des Beschlagens des Spiegels bei den Untersuchungen besteht. Es ist dann erforderlich, die Untersuchung zunächst einmal zu unterbrechen und ein neues Spiegelinstrument zu verwenden bzw. den Spiegel des alten Spiegelinstruments abzuwischen. Ist der Spiegel lediglich leicht beschlagen, so ist in nachteiliger Weise die freie Sicht des behandelnden Arztes eingeschränkt.

Der Erfindung liegt die Aufgabe zugrunde, ein Spiegelinstrument der eingangs beschriebenen Art so weiterzubilden, daß die Gefahr des Beschlagens des Spiegels weitgehend vermieden wird.

Erfindungsgemäß wird dies dadurch erreicht, daß eine Heizvorrichtung vorgesehen ist, und daß die Heizvorrichtung den Spiegel auf insbesondere Körpertemperatur eines Patienten aufheizt. Die Erfindung geht von der Erkenntnis aus, daß das Beschlagen des Spiegels auf den Temperaturunterschied einerseits der auf den Spiegel auftreffenden Luft und andererseits des Spiegels zurückzuführen ist. Vor der Behandlung des Patienten mit dem Spiegelinstrument nimmt der Spiegel, ohne daß die Heizvorrichtung aktiviert wurde, die entsprechende Raumtemperatur an. Bei beispielsweise zahnärztlichen Untersuchungen trifft die Atemluft des Patienten auf den Spiegel. Diese Atemluft hat jedoch eine höhere Temperatur als die Raumtemperatur, so daß die Gefahr des Beschlagens des Spiegels besteht. Wird dagegen mit der Heizvorrichtung der Spiegel vor der Behandlung aufgeheizt, so setzt sich keine Feuchtigkeit an dem Spiegel ab, der Spiegel beschlägt also nicht. Die Temperatur, auf die der Spiegel von der Heizvorrichtung aufgeheizt wird, soll insbesondere der Körpertemperatur des Patienten angeglichen werden, also etwa 37° C betragen. Diese Temperatur liegt etwas oberhalb der Temperatur der Atemluft des Patienten. Dadurch ist einerseits sichergestellt, daß der Spiegel aufgrund der höheren Temperatur nicht mehr beschlägt und andererseits, daß die Behandlung über einen Zeitraum hinweg durchgeführt werden kann, ohne daß die Temperatur des Spiegels unter die der Atemluft des Patienten absinkt.

Die Heizvorrichtung kann eine Wärmespeicher aufweisen. Der Wärmespeicher kann aus beispielsweise Schamotte bestehen, wobei die Schamotte im Innern des Handgriffs angeordnet sein kann. Es ist somit möglich, die einmal gespeicherte Wärme über einen längeren Zeitraum aufrechtzuerhalten, also die Temperatur des Spiegels über einen relativ langen Zeitraum oberhalb der Temperatur der Atemluft des Patienten konstantzuhalten. Die im Innern des Handgriffs angeordnete Schamotte wird beispielsweise in einem Wärmefach aufgeheizt. In dem Schamotte speichert sich dann die Wärme, die während der Behandlung dem Spiegel zugeführt wird. Dafür kann ein wärmeleitendes Material vorgesehen sein, welches die Wärme aus dem Wärmespeicher abführt und dem Spiegel zuführt. Vorzugsweise kann als wärmeleitendes Material ein Kupferdraht vorgesehen sein, der mit seinem einen Ende in dem Wärmespeicher und mit dem anderen Ende unterhalb des Spiegels angeordnet ist. Die aufgeheizte Schamotte gibt dann die Wärme an den Kupferdraht ab, der als wärmeleitendes Material diese bis zu dem Spiegel befördert und diesen dann entsprechend aufheizt. Um den Heizvorgang des Spiegels zu verbessern, kann der Kupferdraht unterhalb des Spiegels in Form einer Spirale vorgesehen sein. Um die Temperatur des Spiegels möglichst lange oberhalb der Temperatur der Atemluft zu halten, sollte der Wärmespeicher möglichst groß ausgelegt sein. Der Wärmespeicher ist jedoch durch die geometrische Gestalt des Handgriffs begrenzt. Um den zur Verfügung stehenden Wärmespeicher möglichst optimal auszunutzen, kann die Wärmeabstrahlung des Wärmespeichers nach außen möglichst vermieden werden. Um dies zu erreichen, kann der Wärmespeicher mit einem Isoliermaterial ummantelt werden. Es hat sich dabei als besonders vorteilhaft erwiesen, wenn die äußere Hülle des Handgriffs aus Isoliermaterial bestehend vorgesehen ist. Dies bietet den Vorteil, daß der Wärmespeicher einerseits möglichst groß ausgebildet sein kann, um eine entsprechende Wärmekapazität zu speichern und andererseits die Wärmeabstrahlung durch das Isoliermaterial weitgehend vermieden wird, wobei für das Isoliermaterial die ohnehin notwendige Hülle des Handgriffs verwendet wird, für das Isoliermaterial also kein weiterer Raum zur Verfügung gestellt werden muß.

In dem Wärmespeicher können Heizelemente vorgesehen sein, die an eine Energiequelle anschließbar sind und den Wärmespeicher aufheizen. Als Energiequelle ist eine elektrische Energiequelle denkbar. Der Anschluß kann über eine Kabelverbindung, die vorzugs am Handgriff angeordnet ist und mittels eines Steckers und einer Buchse lösbar ist, erfolgen, oder aber nach dem Induktionsprinzip. Diese Energiequelle versorgt die Heizelemente des Wärmespeichers, wodurch die Schamotte entsprechend aufgeheizt wird. Es besteht somit die Möglichkeit, auch während des Behandlungsvorgangs durch den entsprechenden Anschluß der Heizelemente an die Energiequelle den Wärmespeicher aufzuladen. Es wäre auch denkbar, daß auf die Speicherung der Wärme weitgehend verzichtet wird und stattdessen ein permanentes Aufheizen der Heizelemente und damit letztendlich des Spiegels die notwendige Temperatur des Spiegels erzeugt.

Dem wärmeleitenden Material kann ein Anschluß für die Energiequelle zugeordnet sein und somit kann das wärmeleitende Material sowohl als Heizelement als auch zum Wärmetransport vorgesehen sein. Das wärmeleitende Material dient also sowohl dem Wärmetransport als auch als Heizelement. Über die Energiequelle wird dem wärmeleitenden Material Wärme zugeführt, die es ggf. an die Schamotte abgibt und dort speichert. Andererseits, insbesondere bei zu kleinem Wärmespeicher, kann der Spiegel auch während der Behandlung aufgeheizt werden, indem das wärmeleitende Material auch während dieser Behandlungszeit mit der Energiequelle verbunden bleibt.

Die Erfindung wird anhand von bevorzugten Ausführungsbeispielen weiter beschrieben. Es zeigen:

Figur 1 eine Draufsicht auf ein Spiegelinstrument und

Figur 2 eine Seitenansicht des Spiegelinstruments.

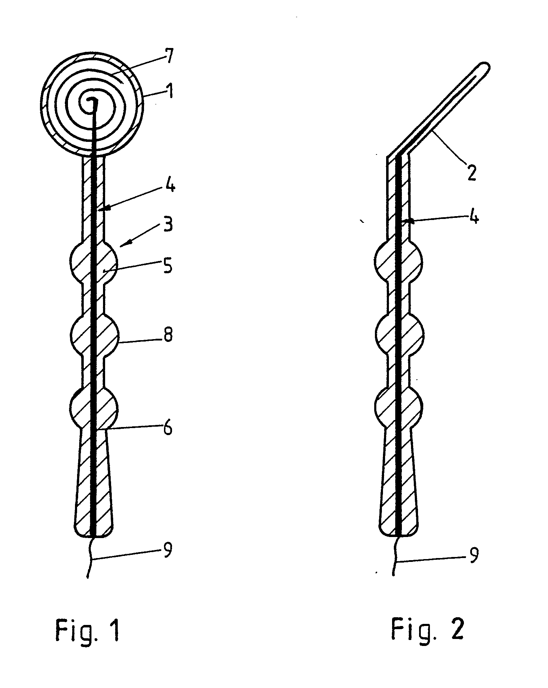

Das Spiegelinstrument entsprechend Figur 1 weist einen runden Träger 1 auf, auf dem ein Spiegel 2 befestigt ist. Der Träger 1 ist mit einem Handgriff 3 fest verbunden. Es besteht auch die Möglichkeit, daß der Träger 1 mit dem Handgriff 3 über eine Schraubverbindung oder einen Bajonettverschluß lösbar verbunden ist. Zwischen dem Träger 1 bzw. dem Spiegel 2 und dem Handgriff 3 ist eine Abwinklung vorgesehen, siehe Figur 2, so daß die Längsachse des Trägers 1 mit der Längsachse des Handgriffs 3 in etwa einen Winkel von 45° bildet. Diese Abwinklung kann aber auch jeden beliebigen anderen Winkel aufweisen oder ganz weggelassen werden, je nachdem, wie es der jeweilige Anwendungsfall erfordert. Es ist eine Heizvorrichtung 4 vorgesehen, die den Spiegel 2 auf eine vorher bestimmte Temperatur aufheizt. Die Heizvorrichtung 4 besteht im wesentlichen aus einem Wärmespeicher 5, der aus Schamotte gebildet ist und im Handgriff 3 angeordnet ist und aus einem wärmeleitenden Material 6, insbesondere Kupferdraht, welches mit seinem einen Ende in den Wärmespeicher 5 hineinragt und mit seinem anderen Ende auf dem Träger 1, unterhalb des Spiegels 2, angeordnet ist. Das wärmeleitende Material 6 ist im Bereich des Trägers 1 in Form einer Spirale 7 gebogen, so daß über den Spiegel 2 hinweg eine möglichst gleichmäßige Temperaturverteilung vorliegt. Um den Wärmespeicher 5 ist die äußere Hülle 8 des Handgriffs 3 vorgesehen, die aus einem wärmeisolierenden Material besteht. An einem hier nur schematisch dargestellten Anschluß 9 kann eine Energiequelle angeschlossen werden, die den Wärmespeicher 5 aufheizt. Der Anschluß 9 kann mit besonderen, hier nicht weiter dargestellten Heizelementen verbunden sein, die ihrerseits wiederum mit dem Wärmespeicher 5 in Verbindung stehen. Es ist jedoch auch möglich, das wärmeleitende Material 6 direkt mit dem Anschluß 9 zu versehen, so daß dieses dann als Heizelement dient. Der Anschluß 9 kann auch in Wegfall kommen, wobei dann die Aufheizung des Wärmespeichers 5 durch vorherige Aufheizung in beispielsweise einem Wärmefach erfolgt.

Vor der Behandlung mit dem Spiegelinstrument ist dieses entweder in dem Wärmefach entsprechend aufzuheizen oder aber es ist die Energiequelle an dem Anschluß 9 zu befestigen. Sobald der Wärmespeicher 5 aufgeladen ist, kann die Energiequelle entfernt werden bzw. das Spiegelinstrument aus dem Wärmefach entnommen werden. Es besteht aber auch die Möglichkeit, die Energiequelle mit dem Anschluß 9 weiterhin in Verbindung zu belassen. Die in dem Wärmespeicher 5 gespeicherte Wärme wird von dem wärmeleitenden Material 6 langsam abgeführt und heizt über die Spirale 7 den Spiegel 2 auf. Dadurch wird ein Beschlagen des Spiegels 2 solange vermieden, bis der Wärmespeicher 5 nicht mehr genügend Kapazität für den Aufheizvorgang hat, die Temperatur des Spiegels 2 also unterhalb derjenigen der Atemluft eines Patienten absinkt. Um solch ein Entladen des Wärmespeichers 5 zu vermeiden, kann das wärmeleitende Material 6 über den Anschluß 9 auch während der Behandlung mit der Energiequelle verbunden bleiben, wodurch der Wärmespeicher 5 kontinuierlich aufgeheizt wird. Die Leistung der Energiequelle ist abhängig von dem jeweiligen Wärmespeicher 5, dem Spiegel 3, dem wärmeleitenden Material 6 und den jeweiligen Einsatzbedingungen. Es ist auf jeden Fall sicherzustellen, daß der Spiegel 2 keine für den Patienten unangenehme Temperatur erreichen kann.

**Bezugszeichenliste:**

1 = Träger
2 = Spiegel
3 = Handgriff
4 = Heizvorrichtung
5 = Wärmespeicher
6 = wärmeleitendes Material
7 = Spirale
8 = äußere Hülle
9 = Anschluß

**Ansprüche**

1. Spiegelinstrument, insbesondere Mundspiegel für zahnärztliche Untersuchungen, mit einem einen Spiegel aufnehmenden, zumeist kreisförmig ausgebildeten Träger und mit einem mit dem Träger befestigten Handgriff, dadurch gekennzeichnet, daß eine Heizvorrichtung vorgesehen ist, und daß die Heizvorrichtung den Spiegel auf insbesondere Körpertemperatur eines Patienten aufheizt.

2. Spiegelinstrument nach Anspruch 1, dadurch gekennzeichnet, daß die Heizvorrichtung einen Wärmespeicher aufweist.

3. Spiegelinstrument nach Anspruch 2, dadurch gekennzeichnet, daß der Wärmespeicher aus beispielsweise Schamotte besteht und daß die Schamotte im Innern des Handgriffs angeordnet ist.

4. Spiegelinstrument nach Anspruch 2 oder 3 , dadurch gekennzeichnet, daß ein wärmeleitendes Material vorgesehen ist, welches die Wärme aus dem Wärmespeicher abführt und dem Spiegel zuführt.

5. Spiegelinstrument nach Anspruch 4, dadurch gekennzeichnet, daß als wärmeleitendes Material ein Kupferdraht vorgesehen ist, der mit seinem einen Ende in dem Wärmespeicher und mit dem anderen Ende unterhalb des Spiegels angeordnet ist.

6. Spiegelinstrument nach Anspruch 5, dadurch gekennzeichnet, daß der Kupferdraht unterhalb des Spiegels in Form einer Spirale vorgesehen ist.

7. Spiegelinstrument nach einem oder mehreren der Ansprüche 2 bis 6, dadurch gekennzeichnet, daß der Wärmespeicher mit Isoliermaterial ummantelt ist.

8. Spiegelinstrument nach Anspruch 7, daduch gekennzeichnet, daß die äußere Hülle des Handgriffs aus Isoliermaterial bestehend vorgesehen ist.

9. Spiegelinstrument nach einem oder mehreren der Ansprüche 2 bis 8, dadurch gekennzeichnet, daß in dem Wärmespeicher Heizelemente vorgesehen sind, die an eine Energiequelle anschließbar sind und den Wärmespeicher aufheizen.

10. Spiegelinstrument nach Anspruch 9, dadurch gekennzeichnet, daß dem wärmeleitenden Material ein Anschluß für die Energiequelle zugeordnet ist und somit das wärmeleitende Material sowohl als Heizelement als auch zum Wärmetransport vorgesehen ist.

Fig. 1

Fig. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-3 428 679 (B. WERNER) * Seite 13, Zeile 15 - Seite 14, Zeile 6; Figuren 1,2 * | 1,2,4,9 | A 61 B 1/24 |
| A | | 5,6,10 | |
| | --- | | |
| A | CH-A- 618 598 (DR. P. J. NYBERG) * Zusammenfassung; Figur; Seite 2, rechte Spalte, Zeilen 7-35 * | 1,2,7 | |
| | --- | | |
| X,P | DE-U-8 808 327 (R. HENSCHEL) * Seite 8, Zeile 13 - Seite 10, Zeile 5; Figuren 1,2 * | 1-10 | |
| | ----- | | **RECHERCHIERTE SACHGEBIETE (Int. Cl.4)** |
| | | | A 61 B 1/00 |

Der vorliegende Recherchenbericht wurde für alle Patentansprüche erstellt

| Recherchenort | Abschlußdatum der Recherche | Prüfer |
|---|---|---|
| BERLIN | 12-09-1989 | WEIHS J.A. |